# EUROPEAN PATENT SPECIFICATION

(11) **EP 2 805 153 B1**
(45) Date of publication and mention of the grant of the patent: **21.12.2016**
(21) Application number: 12866288.9
(22) Date of filing: 19.01.2012
(51) Int. Cl.: G01N 21/65, G01N 21/64, G01N 21/76, G01N 33/04

(54) **MOLECULAR SENSING DEVICE**
MOLEKULARE MESSVORRICHTUNG
DISPOSITIF DE DÉTECTION MOLÉCULAIRE

(43) Date of publication of application: 26.11.2014
(73) Proprietor: Hewlett-Packard Development Company, L.P., Houston, TX 77070 (US)
(72) Inventor: KIM, Ansoon, Palo Alto, CA 94304-1100 (US); LI, Zhiyong, Palo Alto, CA 94304-1100 (US)
(74) Representative: Hufton, David Alan
(86) International application number: PCT/US2012/021889
(87) International publication number: WO 2013/109280

(56) References cited:
- WO-A1-02/074899
- WO-A1-2009/068041
- WO-A1-2011/148179
- US-A- 5 255 067
- US-A1- 2005 148 064
- US-A1- 2007 254 377
- US-A1- 2008 024 776
- US-A1- 2008 270 042

## Description

### BACKGROUND

The present disclosure relates generally to molecular sensing devices.

Assays and other sensing systems have been used in the chemical, biochemical, medical and environmental fields to detect the presence and/or concentration of one or more chemical substances. Some sensing techniques utilize color or contrast for substance detection and measurement, for example, those techniques based upon reflectance, transmittance, fluorescence, or phosphorescence. Other sensing techniques, such as Raman spectroscopy or surface enhanced Raman spectroscopy (SERS), study vibrational, rotational, and other low-frequency modes in a system. In particular, Raman spectroscopy is used to study the transitions between molecular energy states when photons interact with molecules, which results in the energy of the scattered photons being shifted. The Raman scattering of a molecule can be seen as two processes. The molecule, which is at a certain energy state, is first excited into another (either virtual or real) energy state by the incident photons, which is ordinarily in the optical frequency domain. The excited molecule then radiates as a dipole source under the influence of the environment in which it sits at a frequency that may be relatively low (i.e., Stokes scattering), or that may be relatively high (i.e., anti- Stokes scattering) compared to the excitation photons. The Raman spectrum of different molecules or matters has characteristic peaks that can be used to identify the species.

WO 2011/148179 describes methods and apparatus relating to surface-enhanced Raman spectroscopy.

US 5,255,067 describes a substrate and apparatus for surface-enhanced Raman spectroscopy.

WO 2009/068041 relates to a three-dimensional optical structure for surface-enhanced spectroscopy based on surface plasmons.

WO 02/074899 describes enhancing surfaces for optical analyte detection, including Raman spectroscopy.

The present invention is directed to a molecular sensing device as defined in claim 1, to a method of using said molecular sensing device as defined in claim 11, to a surface enhanced Raman spectroscopy (SERS) sensing system comprising said molecular sensing device as defined in claim 12, and to a method for making a molecular sensing device as defined in claim 13.

### BRIEF DESCRIPTION OF THE DRAWINGS

Features and advantages of examples of the present disclosure will become apparent by reference to the following detailed description and drawings, in which like reference numerals correspond to similar, though perhaps not identical, components. For the sake of brevity, reference numerals or features having a previously described function may or may not be described in connection with other drawings in which they appear.
Fig. 1A is a perspective view of an example of a molecular sensing device including a membrane as a molecular selective device;
Fig. 1B is a cross-sectional view taken along line 1 B-1 B of Fig. 1A;
Fig. 2 is a perspective view of another example of the molecular sensing device including the membrane as the molecular selective device;
Fig. 3A is a perspective view of an example of the molecular sensing device including a solid extraction column as the molecular selective device;
Fig. 3B is a cross-sectional view taken along line 3B-3B of Fig. 3A;
Fig. 4 is a perspective view of another example of a molecular sensing device including an array of wells;
Fig. 5 is a schematic illustration of an example of a surface enhanced Raman spectroscopy (SERS) sensing system;
Fig. 6 is a graph depicting the SERS spectra of different concentrations of melamine in water, raw milk, and milk filtered using an example of the molecular sensing device including a membrane as the molecular selective device; and
Fig. 7 is a graph depicting the SERS spectra of different concentrations of melamine in water and infant formula filtered using an example of the molecular sensing device including a membrane as the molecular selective device and using another example of the molecular sensing device including a solid extraction column as the molecular selective device.

### DETAILED DESCRIPTION

Samples to be analyzed via sensing applications (e.g., Raman sensing techniques) may be very complex, with a variety of molecules ranging from those that are of interest and those that are background. It has been found that the background molecules may overwhelm the signal of the molecules of interest, thereby masking the capability to detect the molecules of interest. As an example, a milk sample includes milk proteins, fatty acids, and other large molecular species that may dominate the sample and occupy the sensor surface. These large species may render the detection of other species, such as melamine, difficult and in some instances impossible when the other species is present at a low concentration range (e.g., less than 1 mM).

Examples of the molecular sensing device disclosed herein enable samples to be filtered right before they are introduced into a well of a sensing device that contains a signal amplifying structure. In-line filtering is accomplished via a molecular selective device that is operatively and removably positioned with respect to the well. The molecular selective device is selected to allow molecules of interest to pass through to the well, while filtering out background molecules. This advantageously simplifies the sample solution and enables the sensitive detection of molecules at low concentration levels. Upon completion of filtering, the molecular selective device may be removed to expose the contents of the well for subsequent molecular sensing. The removable integration of the molecular selective device into the molecular sensing device enables sample screening and sensing to be accomplished with a single device.

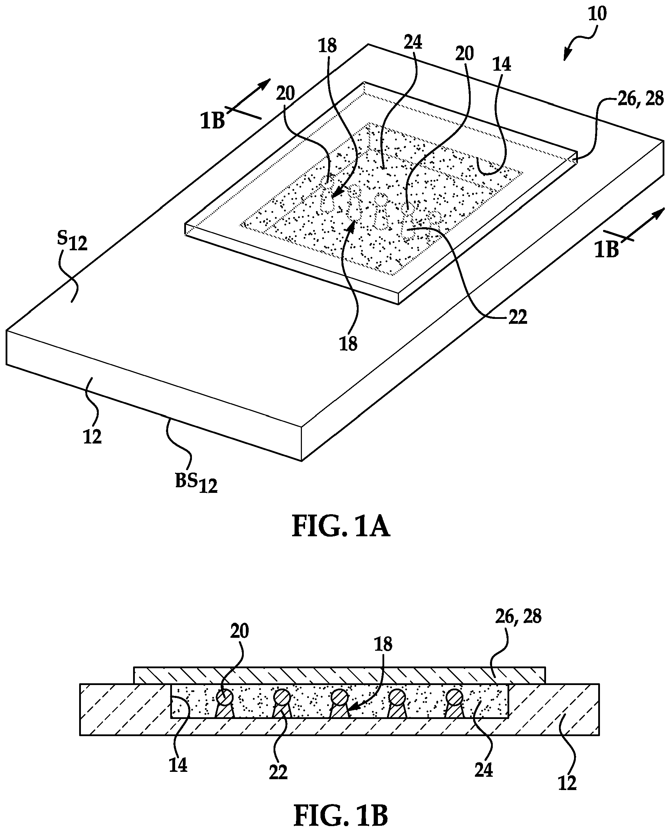

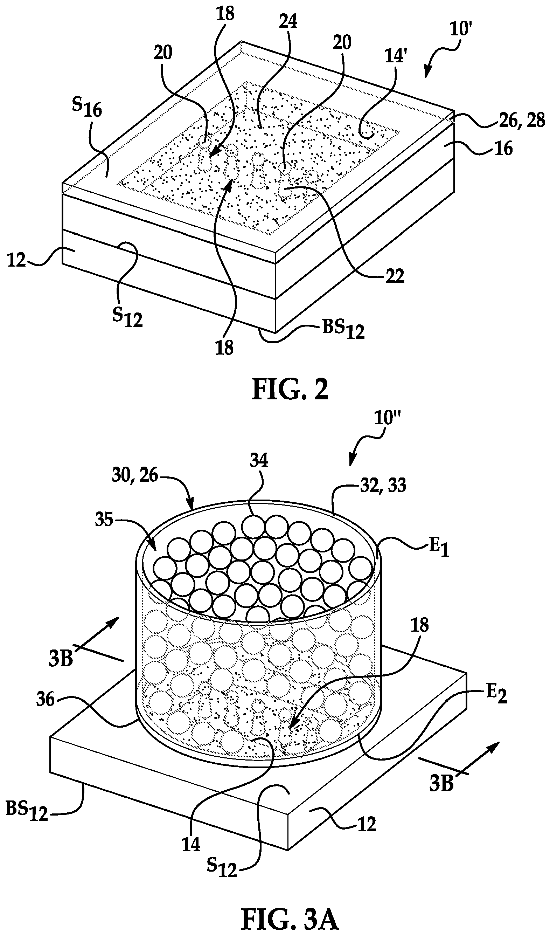

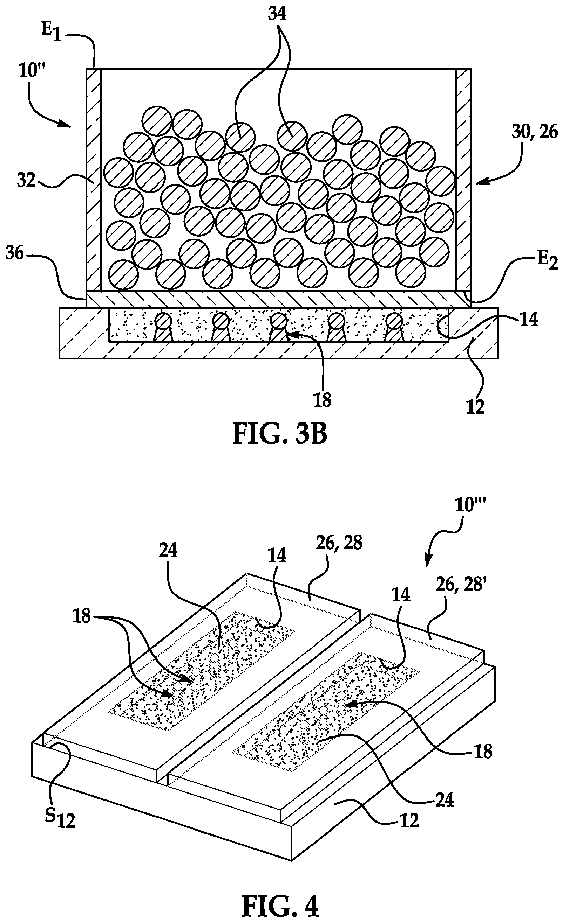

Two examples of the molecular selective device are disclosed herein. In some examples, the molecular selective device is a membrane having a molecular weight threshold. These examples are described in reference to at least Figs. 1A, 1B and 2. In other examples, the molecular selective device is a solid extraction column. These examples are described in reference to at least Figs. 3A and 3B.

Referring now to the examples shown in Figs. 1A, 1B, 2, 3A and 3B, three examples of the molecular sensing device 10, 10' and 10" are respectively depicted. It is to be understood that components that are common to the devices 10, 10' and 10" will be described together, and then the different molecular selective devices of the devices 10, 10', 10" will be described.

Each of the molecular sensing devices 10, 10', 10" includes a substrate 12. The molecular sensing devices 10 and 10" shown in Figs. 1A, 1B, 3A and 3B include a well 14 that is formed in a surface S₁₂ of the substrate 12, while the molecular sensing device 10' shown in Fig. 2 includes a well 14' that is formed in a surface S₁₆ of a material 16 that is positioned on the surface S₁₂ of the substrate 12.

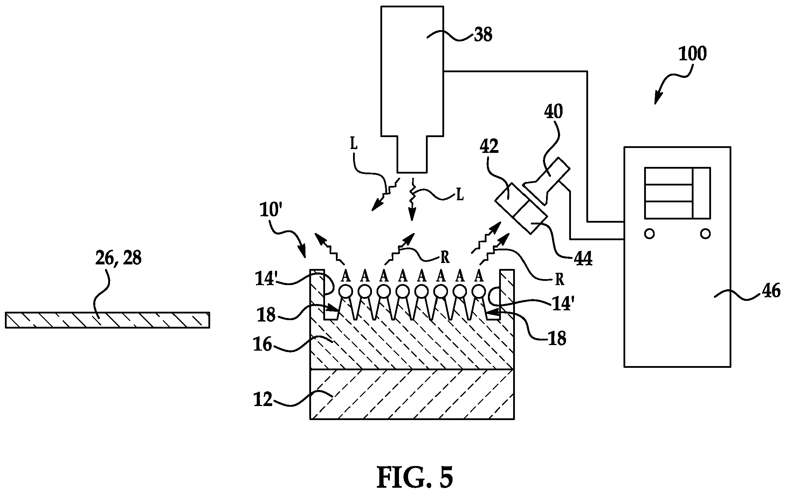

The substrate 12 in any of the examples shown in Figs. 1A, 1B, 2, 3A and 3B may be transparent or reflective, depending upon the position of the molecular sensing device 10, 10', 10" when used in a sensing system (e.g., system 100 shown in Fig. 5). For example, if a sensor is positioned across from the surface S₁₂ or S₁₆ in which the well 14 or 14' is formed, the substrate 12 may be selected from reflective and/or non-reflective materials. Examples of suitable substrates in this example include germanium, silicon, or transparent substrates such as, e.g., glass, quartz, nitrides, alumina, sapphire, indium tin oxide, transparent polymers (e.g., polycarbonate, polyimide, acrylic, etc.), combinations thereof, and/or layers thereof. In an example, the transparent substrate includes a reflective mirror on a back surface BS₁₂ of the substrate 12. However, if a sensor is positioned across from the back surface BS₁₂ (i.e., the surface opposed to the surface S₁₂ or S₁₆ in which the well 14 or 14' is formed), the substrate 12 is selected from transparent materials so that any generated signal (e.g., scattered light) may be transmitted through the substrate 12 to the sensor. Examples of suitable transparent substrates include glass, quartz, nitrides, alumina, sapphire, indium tin oxide, transparent polymers (e.g., polycarbonate, polyimide, acrylic, etc.), or combinations or layers thereof. When the well 14 is formed in the substrate 12, it is to be understood that the substrate 12 may also be selected from a material that is capable of having the well 14 formed therein (e.g., via etching, imprinting, or another suitable technique).

In any of the examples, the substrate 12 may have any desirable dimensions. In an example, the substrate 12 has the dimensions of a test strip that can be inserted into a container (e.g., a beaker) that contains a sample solution. In the examples shown in Figs. 1A and 3A, the substrate 12 may be small enough to fabricate a single well 14 therein or may be large enough to fabricate a plurality of wells 14 therein. The thickness of the substrate 12 shown in Figs. 1A and 3A may be over 1 µm thick so that the well 14 formed therein has a depth that is about 1 µm. In the example shown in Fig. 2, the substrate 12 may be small enough to fabricate a single well 14' in the material 16 positioned thereon, or may be large enough to fabricate a plurality of wells 14' in the material 16 positioned thereon. The substrate 12 shown in Fig. 2 may have any desirable thickness that provides support to the material 16 positioned thereon.

In the example shown in Fig. 2, the material 16 is deposited (and in some instances cured) on the substrate 12 to at least a desirable thickness for the well 14' that is to be formed therein. For example, the thickness of the material 16 may be over 1 µm thick so that the well 14' formed therein has a depth that is about 1 µm. In an example, the material 16 may be any transparent material that is capable of having the well 14' formed therein (e.g., via etching, imprinting, or another suitable technique). Transparent materials 16 may be desirable when the substrate 12 is transparent. Examples of suitable transparent materials include glass, quartz, nitrides, alumina, silica, sapphire, transparent polymers, or combinations thereof. If the substrate 12 is not transparent, the material 16 may or may not be a transparent material. Examples of other suitable materials 16 include silicon, germanium, titanium, oxides of these materials (e.g., silicon oxide), or nitride. When the well 14' is formed in the material 16 via an imprinting technique (described further below), the material 16 may be an ultraviolet or thermally curable resist. Some suitable resists are commercially available from Nanonex Corp., Monmouth Junction, NJ (e.g., NXR-2000 Series and NXR-1000 Series), and NanoLithoSolution, Inc.. San Marcos, CA (e.g., AR-UV-01).

As mentioned above, the molecular sensing devices 10, 10' and 10" include the well 14 or 14' formed in the surface S₁₂ of the substrate 12 or in the surface S₁₆ of the material 16. In the example shown in Figs. 1A, 1B, 3A and 3B, the well 14 is a cavity that extends from the surface S₁₂ of the substrate 12 into the substrate 12 to a desirable depth that is less than the thickness of the substrate 12. In the example shown in Fig. 2, the well 14' is a cavity that extends from the surface S₁₆ of the material 16 into the material 16 to a desirable depth that is less than or equal to the thickness of the material 16. As such, in an example, the well 14 extends through the entire thickness of the material 16 so that the substrate surface S₁₂ is exposed, and in another example, the well 14' extends through less than the entire thickness of the material 16 so that the substrate surface S₁₂ is not exposed.

The wells 14, 14' may be formed to have any desirable shape (e.g., as the well 14, 14' appears from the top view) and may have any desirable dimensions (e.g., length, width, diameter, etc.), each of which depends, at least in part, on the type and number of signal amplifying structures 18 to be formed in the well 14, 14', the number of wells 14, 14' to be formed, the size of the substrate 12, and, in some instances, the size of the material 16. Example top-view shapes include square, rectangular, circular, triangular, oval, elliptical, etc. The examples shown in Figs. 1A, 1B and 2 have a square shape that continues throughout the depth of the wells 14, 14'. The example shown in Figs. 3A and 3B has a rectangular shape that continues throughout the depth of the wells 14, 14'. Alternatively, the walls of the wells 14, 14' may be tapered so that the dimensions of the well 14, 14' increase toward the surface S₁₂ or S₁₆.

While one well 14, 14' is shown in each of Figs. 1A, 1B, 2, 3A and 3B, it is to be understood that any number of wells 14, 14' may be formed in a single substrate 12 or in a single material 16. There is no limit as to the number of wells 14,14' that may be formed, except as dictated by the size of the substrate 12 used. When multiple wells 14, 14' are formed, each well 14, 14' may be spaced far enough apart from each adjacent well 14, 14' so that the wells 14, 14' are fluidly isolated from one another and so that a sensing technique may be performed within a single well 14, 14' if that is so desirable. As an example, the wells 14, 14' in an array may be at least about 1 µm apart, which, in some instances, is about the limit of a laser spot size.

The molecular sensing devices 10, 10' and 10" shown in Figs. 1A, 1B, 2, 3A and 3B also depict the previously mentioned signal amplifying structure(s) 18 positioned in the wells 14, 14'. The type and number of signal amplifying structures 18 that are used may depend, at least in part, upon the type of sensing to be performed with the molecular sensing device 10, 10', 10". For example, if the molecular sensing device 10, 10', 10" is to be used for surface enhanced Raman spectroscopy (SERS), the signal amplifying structure(s) 18 may be nano-structures, which have at least one dimension ranging from about 0.1 nm to about 100 nm and have a height that is less than the depth of the well 14, 14' in which the nano-structures are formed. As an example, the well 14, 14' has a depth of about 1 µm, and the signal amplifying structure(s) 18 have a height of about 500 nm.

Examples of nano-structures include antennas, pillars or nano-wires, poles, flexible columnar or finger-like structures, cone-shaped structures, multi-faceted structures, etc. The SERS signal amplifying structure(s) 18 may be metal or metal-coated plasmonic nano-structures that amplify the Raman scattering from a molecule (i.e., analyte, species of interest, predetermined species) when exposed to laser illumination. The metal or metal-coating is a signal-enhancing material, or a material that is capable of enhancing the signal that is generated during a particular sensing process. In an example, the signal-enhancing material is a Raman signal-enhancing material that increases the number of Raman scattered photons when the molecule (or other species of interest) is located proximate to the signal amplifying structure(s) 18, and when the molecule and material are subjected to light/electromagnetic radiation. Raman signal-enhancing materials include, for example, silver, gold, and copper.

In Figs. 1A, 1B, 2, 3A and 3B, the Raman signal-enhancing material is labeled 20, and is the material present at the tip of the base portion 22 of the signal amplifying structure(s) 18. When the signal amplifying structure(s) 18 are partially or fully coated with the Raman signal-enhancing material 20, the base portion 22 of the signal amplifying structure(s) 18 may be formed of any other suitable material, such as the substrate 12 material, the material 16, or the like.

The signal amplifying structures 18 may also be configured for use in techniques, such as enhanced fluorescence (e.g., metal-enhanced fluorescence or surface enhanced fluorescence (SEF)) or enhanced chemiluminescence. As an example, for metal-enhanced fluorescence applications, the bases 22 of the signal amplifying structures 18 may be coated with silver nanoparticles. As another example, for enhanced fluorescence applications, the signal amplifying structures 18 may be configured to couple the localized and propagating surface plasmons.

Any number of signal amplifying structure(s) 18 may be present in the well 14, 14', depending, at least in part, upon the dimensions of the well 14, 14', the size of the signal amplifying structure(s) 18, and the type of sensing to be performed. As examples, a single signal amplifying structure 18 may be present in a single well 14, 14', or a single well 14, 14' may include a multi-structure assembly, such as a dimer (i.e., 2 structures 18), trimer (i.e., 3 structures 18), tetramer (i.e., 4 structures 18), pentamer (i.e., 5 structures 18), etc.

The molecular sensing devices 10, 10', 10" shown in Figs. 1A, 1B, 2, 3A and 3B may be formed via a number of methods. In particular, the well 14, 14' may be formed via any suitable technique, which depends, at least in part, upon the type of substrate 12 or material 16 used, and whether it is desirable to sequentially or simultaneously form the well(s) 14, 14' and signal amplifying structure(s) 18.

In some example methods, the signal amplifying structure(s) 18 and well(s) 14, 14' are formed sequentially. In one example method, a two-step masking and etching process may be used. When it is desirable that the well(s) 14 be formed in the substrate 12, this example of the method includes first forming the signal amplifying structure(s) 18 in the substrate 12 and then forming the well(s) 14 in the substrate 12. For example, a mask that provides the desired pattern for the signal amplifying structure(s) 18 may be placed on the substrate 12 and etching may be performed to a desired depth that is less than the thickness of the substrate and less than or equal to the desired depth for the well(s) 14. While the etchant used will depend upon the substrate material that is being used, this step will generally involve an isotropic (wet or dry) etching process. After the signal amplifying structure(s) 18 are formed, the mask will be removed.

Another mask that provides the desired pattern for the well(s) 14 while protecting the previously formed signal amplifying structure(s) 18 may be placed on the substrate 12. Etching is then performed. This etching step may be performed to a desired depth that is less than the thickness of the substrate 12 and less than or equal to the height of the previously formed signal amplifying structure(s) 18. While the etchant used will again depend upon the substrate material that is being used, this step may involve either an isotropic or anisotropic (wet or dry) etching process.

This same sequential process may also be performed in the material 16 so that the well(s) 14' are created in the material 16 and have signal amplifying structure(s) 18 formed therein. It is to be understood that the substrate 12 or material 16 may also have two layers, where the top layer material and thickness is suitable for forming the signal amplifying structure(s) 18 (using the first masking/etching step described above), and the bottom layer material and thickness is suitable for having the well(s) 14, 14' formed therein (using the second masking/etching step described above).

In another example method, the signal amplifying structure(s) 18 and well 14, 14' are formed simultaneously. This may be accomplished using a mold that includes a pattern for both the signal amplifying structure(s) 18 and the well 14 or 14'. The mold may be formed of single crystalline silicon, polymeric materials (acrylics, polycarbonates, polydimethylsiloxane (PDMS), polyimide, etc.), metals (aluminum, copper, stainless steel, nickel, alloys, etc.), quartz, ceramic, sapphire, silicon nitride, or glass.

The pattern in the mold may be for forming a single well 14, 14' with one or more signal amplifying structure(s) 18 therein, or multiple wells 14, 14', each of which has one or more signal amplifying structures 18 therein. The pattern is a negative replica of the desired signal amplifying structure(s) 18 and well 14, 14', and thus defines the shapes for at least the base(s) 22 of the signal amplifying structure(s) 18 and for the well 14, 14' that are to be formed. When more than one signal amplifying structure 18 is desired, the pattern for the signal amplifying structures 18 may all be the same (e.g., all pillars), may all be different (e.g., one pillar, one pole, one finger-like structure, etc.), or the pattern for some the signal amplifying structures 18 may be different from one or more others of the signal amplifying structures 18 (e.g., one pillar, two poles, two cones, etc.). Furthermore, when more than one signal amplifying structure 18 is desired, the pattern for the signal amplifying structures 18 may have the same or different dimensions. Still further, when multiple wells 14, 14' are formed using the same mold, the pattern for the wells 14, 14' may be the same or different for at least one of the wells 14, 14' and the pattern for the signal amplifying structures 18 may be the same or different for at least one of the wells 14, 14'.

The pattern may be integrally formed in the mold, for example, via deep reactive ion etching and passivation. More specifically, the Bosch process may be used, and this process involves a series of alternating cycles of etching (e.g., using SF₆ and O₂ plasmas) and passivation (e.g., using a C₄F₈ plasma). The morphology of the resulting pattern may be controlled by controlling the conditions (e.g., vacuum pressure, RF power, total processing time, individual etching cycle time, individual passivation cycle time, and gas flow rates) of the process. In an example, the etcher may be operated at a pressure of 15 mTorr, the coil and platen powers of the etcher are 800 W and 10 W, respectively, each etching cycle (with SF₆ and O₂) is 6 seconds, each passivation cycle (with C₄F₈) is 5 seconds, and the flow rates for SF₆, O₂, and C₄F₈ are 100 sccm, 13 sccm, and 100 sccm, respectively. More generally, the flow rate may be any rate up to about 100 sccm.

The portion of the pattern that forms the signal amplifying structure(s) 18 may include a regular or non-regular array of the signal amplifying structure shapes. The etching and passivation process previously described often results in a non-regular array. It is to be understood that in order to generate a regular array, a fabrication method, such as focused ion-beam, e-beam lithography, or optical lithography may be used. It is believed that the portion of the pattern that forms the signal amplifying structure(s) 18 may be designed in a predetermined manner to enable the resulting signal amplifying structure(s) 18 to be sensitive to a targeted range on the Raman spectrum (e.g., capable of producing stronger signals in a particular wavelength).

The mold is pressed into the substrate 12 or the material 16. Alternatively, the substrate 12 or material 16 can be deposited on the mold. While the mold is pressed into (or otherwise in contact with) the substrate 12 or material 16, the structure may be exposed to UV light or heat in order to partially or fully cure the substrate 12 or material 16. It is to be understood that the time for UV or heat exposure, the power of the UV lamp used, the temperature of the heat, and other like curing parameters will depend, at least in part, on the substrate 12 or material 16 that is used. Once curing is complete, the mold may be removed, and the resulting structure includes the substrate 12 or material 16 patterned to form the well 14, 14' and the base(s) 22 of the signal amplifying structure(s) 18. In another example, while the mold is pressed into (or otherwise in contact with) the substrate 12 or material 16, partial curing may be performed. Partial curing cures some, but not all, of the substrate 12 or material 16. After partial curing, the mold may be removed. Once the mold is removed, curing may be continued until the substrate 12 or material 16 is fully cured.

In this example method, the signal-enhancing material 20 may then be deposited on at least a surface of the base portion 22 of the signal amplifying structure(s) 18. The signal-enhancing material 20 may be established by any suitable deposition or other coating technique. In some examples, a blanket deposition technique may be used so that the material 20 is established on all of the exposed portions of the substrate or material 16. In other examples, a selective deposition technique may be used so that the material 20 is established on, for example, the tips of the bases 22 alone. As examples, the material 20 may be deposited via electron-beam (e-beam) evaporation or sputtering. In still other examples, the signal-enhancing material 20 can be pre-formed nanoparticles (e.g., of silver, gold, copper, etc.), which are coated onto the substrate 12 or material 16. Such nanoparticles may have an average diameter ranging from about 1 nm to about 10 nm. It is believed that the presence of the material 20 nanoparticles (rather than a continuous coating of material 20) at the apex of the base 22 further enhances the electric field during, e.g., a SERS operation. The material 20 itself may also have a surface roughness that spontaneously forms during the deposition process. This surface roughness can act as additional optical antennas to increases the SERS-active sites over each signal amplifying structure 18.

In still another example method, the well 14, 14' may be formed in the substrate 12 or material 16 using masking and etching or imprinting, and the signal amplifying structures 18 may be formed on a separate web or wafer using masking and etching or imprinting. The signal amplifying structures 18 could then be placed into the well 14, 14'. In this example, the signal amplifying structures 18 may be secured to the well 14, 14' via a suitable adhesive.

Referring now specifically to Figs. 1A, 1B and 2, after the complete signal amplifying structures 18 are formed, an inert fluid 24 may be deposited or otherwise introduced into the well 14, 14'. The inert fluid 24 may be deposited/dispensed, e.g., using any dispenser that is capable of depositing/dispensing amounts of the inert fluid 24 will surround the signal amplifying structure(s) 18, and in some instances, that will fill the well 14, 14'. The inert fluid 24 may be dispensed manually, for example, via tips or pipettes. The inert fluid 24 may also or alternatively be dispensed automatically, for example, via a jet dispenser (e.g., thermal jet dispensers, piezo jet dispensers, piezo-capillary jet dispensers) or an acoustic dispenser (e.g., Labcyte Echo acoustic dispensers). When a gas is used, the structure may be placed into a box or container that is filled with the desired gas.

The inert fluid 24 may be any suitable liquid or gas that will not deleteriously affect (e.g., degrade, change the morphology of, etc.) the signal amplifying structure(s) 18. Examples of suitable inert fluids include neon gas, argon gas, nitrogen-based inert gases, dry air, deionized water, or the like. The inert fluid 24 may be used to prevent the signal amplifying structure(s) 18 from absorbing undesirable species from the ambient environment prior to the introduction of the sample to be tested. When multiple finger-like structures are used as the signal amplifying structures 18 and are included in the same well 14, 14', it is also believed that the inert fluid 24 prevents the finger-like signal amplifying structures 18 from prematurely and irreversibly collapsing toward one another (e.g., due to external forces, such as micro-capillary forces). As such, the inert fluid 24 may also provide stability to the signal amplifying structures 18 while the device 10, 10', or 10" is on-the-shelf.

All of the example devices 10, 10', 10" disclosed herein include a molecular selective device 26. The molecular selective device 26 operates to isolate molecule(s) of interest within a sample from other molecule(s) within the sample that may interfere with sensing of the molecule(s) of interest.

The examples of the devices 10, 10' shown in Figs. 1A, 1B and 2 include a membrane 28 as the molecular selective device 26. The membrane 28 has a molecular weight (in atomic mass units, amu) threshold or cutoff, where molecules with a molecular weight above the threshold or cutoff are unable to migrate through the membrane 28. Similarly, those molecules that have a molecular weight at or below the threshold or cutoff are able to migrate through the membrane 28. Examples of membrane 28 include cellulose membranes, or porous inorganic membranes having a thickness equal to or less than 100 µm. In an example, thickness of the porous inorganic membrane is equal to or less than 50 µm. Suitable porous inorganic membranes may be formed of glass or ceramic materials.

As shown in Figs. 1A, 1B and 2, the membrane 28 is positioned over the well 14, 14'. In these examples, the membrane 28 covers the entire opening to the well 14, 14' and at least a portion of the surface S₁₂ or S₁₆ of the substrate 12 or material 16 surrounding the well 14, 14'. The membrane 28 may either be placed into position over the well 14, 14' when it is desirable to introduce a sample into the well 14, 14', or it may be removably bonded to the areas of the substrate 12 or material 16 that are adjacent to the well 14, 14'. When removably bonded, a suitable adhesive may be used between the substrate 12 or material 16 and the membrane 28. The adhesive selected may be one that enables the membrane 28 to be peeled off of the substrate 12 or material 16 after the sample has been introduced and before a sensing technique is performed. A variety of epoxies that can hermetically seal the membrane 28 are available from Epoxy Technology Inc., Billerica, MA. Some adhesives will form a robust bond between the membrane 28 and the substrate 12, and in these instances, the membrane 28 is not peelable. In these instances, a mechanical tool (e.g., sharp tip, razor blade, etc.) may be used to poke through the membrane 28 to expose the single amplifying structure 18.

When the membrane 28 is utilized, select molecules of a sample containing multiple molecules may be introduced into the well 14, 14' through membrane 28 in a variety of different ways. In an example, the device 10, 10' (having the membrane 28 secured thereto) may be immersed into the sample, and molecules at or below the threshold of the membrane 28 will diffuse through the membrane 28 and into the well 14, 14'. In another example, the sample may be poured on, dispensed on, other otherwise introduced to the surface of the membrane 28 so that molecules at or below the threshold of the membrane 28 will diffuse through the membrane 28 and into the well 14, 14'.

The example of the device 10" shown in Figs. 3A and 3B includes a solid extraction column 30 as the molecular selective device 26. The solid extraction column 30 includes a housing 32, a molecular extraction material 34 packed within the housing 32, and a porous membrane 36 connected to the housing 32 at one of two opposed ends E₁, E₂.

The housing 32 includes one or more walls 33 that may be formed of a polymeric material (e.g., polyproprene, polyethylene terephthalate (PET), polyetherketone (PEEK), polycarbonate, etc.), a glass material, or a metal material (e.g., aluminum). The housing wall(s) 33 define a perimeter of the housing 32 and the space 35 within the perimeter is hollow. The housing 32 is shaped so that when the column 30 is in an operable position with respect to the well 14, 14', the housing wall(s) 33 surround the well 14, 14'. The housing 32 may have the same shape (from a top view) as the well 14, 14', or as shown in Fig. 3A, may have a different shape than the well 14, 14'. The wall(s) 33 may form a hollow cylinder with two open opposed ends, a hollow cube with two open opposed ends, a hollow rectangular prism with two open opposed ends, or any other geometry that can define a hollow structure having two open opposed ends.

Packed within the space 35 of the housing 32 is the molecular extraction material 34. The molecular extraction material 34 allows molecules with different properties to pass through the solid extraction column 30 at different speeds. More particularly, molecules that have a smaller binding coefficient with the extraction material 34 will pass through the column before those molecules that have a larger binding coefficient with the extraction material 34. At least some of the molecules with the larger binding coefficient will not pass through the entire column 30 because these molecules attach to the surface(s) of the molecular extraction material 34. Examples of molecular extraction materials 34 include cross-linked polymer spheres, silica spheres, glass spheres, alumina spheres, polystyrene spheres, functionalized spheres, or combinations thereof. An example of the cross-linked polymer spheres is cross-linked dextrans. It is believed that other cross-linked polymers may be used as well. Functionalized spheres are any of the previously mentioned spheres (e.g., silica, alumina, polystyrene, etc.) having a ligand or another anchor species (e.g., an amine, a thiol, a carboxyl, ethylene glycol, silanol, DNA, proteins, etc.) that will enhance the specific binding of the molecules with the larger binding coefficient.

The amount of molecular extraction material 34 used will depend upon the size of the space 35 defined by the housing 32. In some examples, the extraction material 34 in the housing 32 may be a macroscale column that ranges from about 2 mm long up to tens of cm long. In other examples, the extraction material 34 in the housing 32 may be a microscale column. In these examples, a very small amount of the extraction material 34 beads or spheres may be packed inside a microfluidic channel (having a width and/or depth ranging from about 10 µm to 900 µm, and a length ranging from about 1 mm to about 1 cm) on a microfluid-on-a-chip platform.

The housing walls 33 define the two opposed ends E₁, E₂ of the housing 32. Attached to one of these ends E₁, E₂ (e.g., end E₂ in Figs. 3A and 3B) is the porous membrane 36. When the column 30 is in an operable position with respect to the well 14, 14', the porous membrane 36 is positioned between the housing 32 and the substrate or material surface S₁₂, S₁₆, and between the well 14, 14' and the molecular extraction material 34. The porous membrane 36 maintains the molecular extraction material 34 within the housing 32 while allowing the molecules that pass through the housing 32 and past the molecular extraction material 34 to migrate through to the well 14, 14'. The porous membrane 36 may be formed of any porous material that allows the desired molecule migration from the column 30 to the well 14, 14'. Examples of the porous membrane 36 include organic materials (e.g., regenerated cellulose, cellulose ester, resins, etc.) or inorganic materials (e.g., sintered glass, ceramics, etc.) having a thickness equal to or less than 100 µm.

As shown in Figs. 3A and 3B, the solid extraction column 30 is positioned so that the molecular extraction material 34 overlies the well 14, 14'. In these examples, the solid extraction column 30 covers the entire opening to the well 14, 14' and at least a portion of the surface S₁₂ or S₁₆ of the substrate 12 or material 16 surrounding the well 14, 14'. The solid extraction column 30 may either be placed into position over the well 14, 14' when it is desirable to introduce a sample into the well 14, 14', or it may be removably bonded to the areas of the substrate 12 or material 16 that are adjacent to the well 14, 14'. When removably bonded, a suitable adhesive may be used between the substrate 12 or material 16 and the porous membrane 36. The adhesive selected may be one that enables the solid extraction column 30 to be removed from the substrate 12 or material 16 after the sample has been introduced and before a sensing technique is performed.

When the solid extraction column 30 is utilized, a sample is introduced into the column 30 through the opening to the space 35 at the end E₁. Sample introduction may be accomplished via pouring, pipetting, dispensing, or some other suitable technique. The molecules within the sample will either pass by the molecular extraction material 34 and through porous membrane 36 into the well 14, 14', or will attach to the surfaces of the molecular extraction material 34 (and thus will not enter the well 14, 14').

Referring now to Fig. 4, another example of the molecular sensing device 10'" is depicted. This device 10'" includes an array of wells 14, 14', each of which is associated with a respective molecular selective device 26. In Fig. 4, the respective molecular selective devices 26 are membranes 28 and 28'. While two membranes 28, 28' are shown, it is to be understood that the molecular selective devices 26 in an array could each be a solid extraction column 30, or some of the molecular selective devices 26 in an array could be membranes 28 while other molecular selective devices 26 in the same array could be solid extraction columns 30.

In this example, the membranes 28, 28' are separately attached to the respective areas of the substrate 12 so that the membranes 28, 28' may be individually removed from the substrate 12. In an example, one membrane 28 or 28' and the corresponding well 14 may be used at a time. In this example, a sample may be filtered through one of membranes 28 into the corresponding well 14 while the other membrane 28' and well 14 remain unused. In another example, each of the membranes 28, 28' and corresponding wells 14 may be used at the same time so that molecules are introduced into each well 14 and a sensing technique is performed using each of the wells 14 simultaneously. A single membrane 28 could also cover both wells 14.

The device 10'" of Fig. 4 is similar to the device 10 shown in Figs. 1A and 1B, at least in part because the wells 14 are formed in the substrate 12. However, the device 10'" could be made similar to the device 10' shown in Fig. 2 by forming the wells 14' in the material 16 present on the substrate 12.

In the example device 10'" shown in Fig. 4, the signal amplifying structures 18 in the two wells 14 are different. One of the wells 14 (on the left side of the figure) contains four finger-like signal amplifying structures 18, and the other of the wells 14 (on the right side of the figure) contains three cone-shaped signal amplifying structures 18. It is to be understood that a single mold may be used to fabricate such wells 14 and structures 18.

Referring now to Fig. 5, an example of a molecular sensing system 100 is depicted. The system 100 includes an example of the device 10, 10', 10" or 10"'. As illustrated, the device 10' is shown after molecules (labeled A) of a sample have been filtered through the molecular selective device 26 and into the well 14, 14', and after the molecular selective device 26 has been removed. In this example, the signal amplifying structures 18 are SERS signal amplifying structures, each of which includes a pillar or a finger-like base 22 and a Raman signal-enhancing material 20 deposited at the tip/top of the base 22.

The system 100 shown in Fig. 5 is a SERS system that includes Raman spectrometer or reader. The Raman reader includes a laser source 38 and a photodetector 40.

The laser source 32 may be a light source that has a narrow spectral line width, and is selected to emit monochromatic light beams L within the visible range or within the near-infrared range. The laser source 32 may be selected from a steady state laser or a pulsed laser. The laser source 32 is positioned to project the light L onto the molecular sensing device 10'. A lens (not shown) and/or other optical equipment (e.g., optical microscope) may be used to direct (e.g., bend) the laser light L in a desired manner. In one example, the laser source 32 is integrated on a chip. The laser source 32 may also be operatively connected to a power supply (not shown).

During operation of the system 100, a sample is introduced to the molecular selective device 26 and is allowed to be filtered by the molecular selective device 26 for a desirable amount of time. The exposure time may range anywhere from 1 second to 10 minutes. Some exposure times may be longer or shorter depending upon the sample used, the device 26 used, etc. After the molecules A are present in the well 14, 14', the molecular selective device 26 is removed. It is to be understood that any inert fluid 24 present within the well 14, 14' may be removed prior to introduction of molecules A or may remain in the well 14, 14' when the molecules A are introduced. Removal of the inert fluid 24 may be accomplished by pouring the liquid 24 out of the well(s) 14, 14' (e.g., before positioning the molecular selective device 26 thereon), by pipetting or suctioning the liquid 24 out of the well(s) 14, 14' (e.g., before positioning the molecular selective device 26 thereon), by gas-flowing through the well(s) 14, 14', by evaporating the liquid 24 from the well(s) 14, 14', or by any other suitable technique.

The molecules A that are filtered through the molecular selective device 26 may settle on a surface of the SERS signal amplifying structures 18 due to gravitational, micro-capillary, and/or chemical forces.

The laser source 32 is then operated to emit light L toward the signal amplifying structures 18. When an array is used, it is to be understood that the entire array of wells 14, 14' (and structures 18 therein) may be exposed at the same time, or one or more individual wells 14, 14' (and structures 18 therein) may be exposed at a particular time. As such, simultaneously sensing or parallel sensing may be performed. The analyte molecules A concentrated at or near the SERS signal amplifying structures 18 of the molecular sensing device 10' interact with and scatter the light/electromagnetic radiation L (note that the scattered light/electromagnetic radiation is labeled R). The interactions between the molecules A and the SERS signal-enhancing material 20 of the SERS signal amplifying structures 18 cause an increase in the strength of the Raman scattered radiation R. The Raman scattered radiation R is redirected toward the photodetector 40, which may optically filter out any reflected components and/or Rayleigh components and then detect an intensity of the Raman scattered radiation R for each wavelength near the incident wavelength.

A processor 46 may be operatively connected to both the laser source 38 and the photodetector 40 to control both of these components 38, 40. The processor 46 may also receive readings from the photodetector 40 to produce a Raman spectrum readout, the peaks and valleys of which are then utilized for analyzing the analyte molecules A. While not shown, the Raman reader may also include the previously mentioned power source (e.g., a battery, plug, etc.) and a data I/O (input and output) display.

The system 100 may also include a light filtering element 42 positioned between the molecular sensing device 10' and the photodetector 40. This light filtering element 42 may be used to optically filter out any Rayleigh components, and/or any of the Raman scattered radiation R that is not of a desired region. The system 100 may also include a light dispersion element 44 positioned between the molecular sensing device 10' and the photodetector 40. The light dispersion element 44 may cause the Raman scattered radiation R to be dispersed at different angles. The elements 42 and 44 may be part of the same device (e.g., the Raman reader) or may be separate devices.

To further illustrate the present disclosure, examples are given herein. It is to be understood that these examples are provided for illustrative purposes and are not to be construed as limiting the scope of the present disclosure.

### EXAMPLE 1

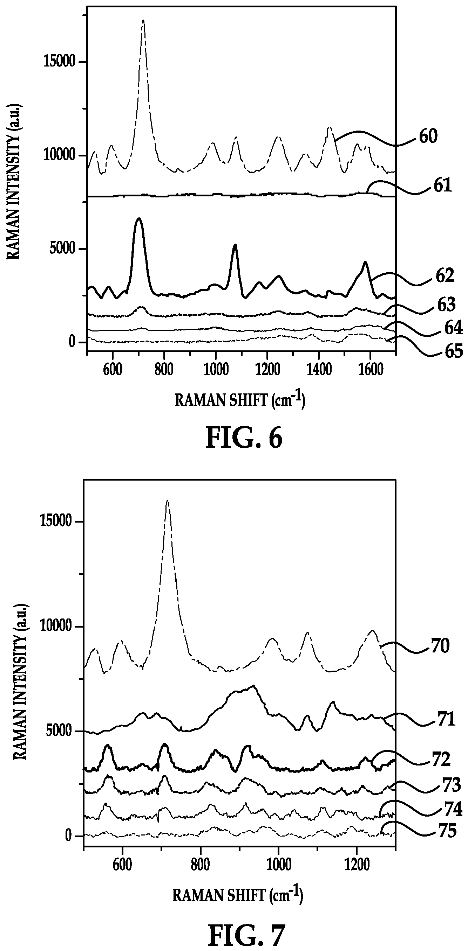

Examples of the molecular sensing device were made in the form of test strips including a molecular size selective membrane made of regenerated cellulose as the molecular selective device. The SERS structures in the well of the test strips were a pentamer of gold nano-finger structures. The test strips were dipped into milk samples containing different concentrations of melamine. Milk sample 1 (labeled 62 in Fig. 6) was spiked with 100 ppm melamine, milk sample 2 (labeled 63 in Fig. 6) was spiked with 10 ppm melamine, milk sample 3 (labeled 64 in Fig. 6) was spiked with 1 ppm melamine, and milk sample 4 (labeled 65) was not spiked with any melamine. The respective strips remained in the respective sample for about 10 minutes in order to allow the smaller molecules (e.g., melamine) to diffuse through the membrane into the well of the molecular sensing device. After the time period, the strips were removed from the respective samples and the membranes were removed. Surface enhanced Raman spectroscopy of the molecules within the wells was performed.

For comparative examples, a molecular sensing device that did not include the molecular selective device was also tested. In the comparative examples, water (labeled 60 in Fig. 6) and raw milk (labeled 61 in Fig. 6, containing 100 ppm melamine) were respectively introduced into the well and surface enhanced Raman spectroscopy was performed.

The SERS spectra of the comparative water sample (60), the comparative raw milk sample (61), and the filtered milk samples (62-65) are shown in Fig. 6. The melamine peaks are at about 710 cm⁻¹. As depicted, the other components that were present in the comparative raw milk sample (61) shielded the SERS amplifying structures in the well of the molecular sensing device and melamine is not detected. The melamine signal was detected in each of the samples (62-64) filtered using the test strip including the molecular size selective membrane, except for the sample including 0 ppm melamine (65). The signal intensity of the 10 ppm sample (63) and 1 ppm sample (64) is not as pronounced due to the scale used in Fig. 6. The SERS intensity of the melamine peak increased as the concentration of spiked melamine in milk was increased. The detection limit of melamine using the molecular size selective membrane of this example was 1 ppm in the milk sample.

### EXAMPLE 2

Examples of the molecular sensing device were made in the form of test strips including a solid extraction column as the molecular selective device. The SERS structures in the well of the test strips were a pentamer of gold nano-finger structures. The solid extraction column was based on gel filtration chromatography. More particularly, the column included a polyethylene housing packed with cross-linked dextran based materials having a desirable pore size for filtering melamine.

Infant formula samples containing different concentrations of melamine were introduced into the columns. Infant formula sample 1 (labeled 72 in Fig. 7) was spiked with 1 ppm melamine, infant formula sample 2 (labeled 73 in Fig. 7) was spiked with 0.5 ppm melamine, infant formula sample 3 (labeled 74 in Fig. 7) was spiked with 0.1 ppm melamine, and infant formula sample 4 (labeled 75 in Fig. 7) was not spiked with any melamine. The columns having the respective samples therein remained in place over the respective wells for about 1 minute in order to allow the smaller molecules (e.g., melamine) to pass through the columns into the wells of the molecular sensing device. After the time period, the columns were removed. Surface enhanced Raman spectroscopy of the molecules within the wells was performed.

A molecular sensing device from Example 1 was also used to test the 1 ppm melamine sample of infant formula. This sample is labeled 71 in Fig. 7.

For comparative examples, a molecular sensing device that did not include the molecular selective device was also tested. In the comparative examples, water was introduced into the well and surface enhanced Raman spectroscopy was performed. This comparative sample is labeled 70 in Fig. 7.

The SERS spectra of the comparative water sample (70), the membrane filtered infant formula sample (71), and the column filtered infant formula samples (72-75) are shown in Fig. 7. The melamine peaks are at about 710 cm⁻¹. As depicted, the other components that were present in the membrane filtered infant formula (71) caused some difficulty in verifying the melamine SERS signal. However, the melamine signal was detected in each of the samples (72-74) filtered using the test strip including the molecular extraction column, except for the sample including 0 ppm melamine (75). The SERS intensity of the melamine peak increased as the concentration of spiked melamine in infant formula was increased. The detection limit of melamine using the column was 0.1 ppm in the formula samples.

It is to be understood that the ranges provided herein include the stated range and any value or sub-range within the stated range. For example, a range from about 0.1 nm to about 100 nm should be interpreted to include not only the explicitly recited limits of about 0.1 nm to about 100 nm, but also to include individual values, such as 0.2 nm, 0.7 nm, 15 nm, etc., and sub-ranges, such as from about 0.5 nm to about 50 nm, from about 20 nm to about 40 nm, etc. Furthermore, when "about" is utilized to describe a value, this is meant to encompass minor variations (up to +/- 10%) from the stated value.

In describing and claiming the examples disclosed herein, the singular forms "a", "an", and "the" include plural referents unless the context clearly dictates otherwise.

While several examples have been described in detail, it will be apparent to those skilled in the art that the disclosed examples may be modified. Therefore, the foregoing description is to be considered non-limiting.

## Claims

1. A molecular sensing device (10), comprising:
a substrate (12);
a well (14) i) formed in a material (16) that is positioned on a surface (S₁₂) of the substrate (12) or ii) formed in a surface (S₁₂) of the substrate (12);
a signal amplifying structure (18) positioned in the well the signal amplifying structure (18) to amplify a signal for one of: surface enhanced Raman spectroscopy; enhanced fluorescence; and enhanced chemiluminescence ; and
a molecular selective device (26) removably positioned in operative contact with the well (14) the molecular selective device (26) configured to allow molecules of interest to pass to the well and to filter out background molecules.

2. The molecular sensing device (10) as defined in claim 1 wherein the molecular selective device (26) is a membrane (28) having a molecular weight threshold.

3. The molecular sensing device (10) as defined in claim 2 wherein the membrane (28) is chosen from a cellulose membrane and a porous inorganic membrane having a thickness equal to or less than 100 µm.

4. The molecular sensing device (10) as defined in claim 2 wherein the membrane (28) is removably bonded to areas of the substrate (12) or the material (16) adjacent to the well (14).

5. The molecular sensing device (10) as defined in claim 1 wherein the molecular selective device (26) is a solid extraction column (30) including:
a housing (32); and
a molecular extraction material (34) packed within the housing (32).

6. The molecular sensing device (10) as defined in claim 5, further comprising a porous membrane (36) connected to the housing (32) to maintain the molecular extraction material (34) within the housing (32).

7. The molecular sensing device (10) as defined in claim 5 wherein the molecular extraction material (34) is chosen from cross-linked polymer spheres, silica spheres, glass spheres, alumina spheres, polystyrene spheres, functionalized spheres, or combinations thereof.

8. The molecular sensing device (10) as defined in claim 1, further comprising an inert fluid (24) incorporated into the well (14).

9. The molecular sensing device (10) as defined in claim 1 wherein the signal amplifying structure (18) is a Raman spectroscopy enhancing structure.

10. The molecular sensing device (10) as defined in claim 1 wherein the device includes:
an array of discrete wells (14) i) formed in the material (16) that is positioned on the surface (S₁₂) of the substrate (12) or ii) formed in the surface (S₁₂) of the substrate (12);
a respective signal amplifying structure (18) in each of the discrete wells (14); and
a respective molecular selective device (26) removably positioned in operative contact with each of the discrete wells (14).

11. A method for using the molecular sensing device (10) as defined in claim 1, the method comprising:
when the molecular selective device (26) is positioned in operative contact with the well (14), exposing the molecular selective device (26) to a sample to introduce components filtered through the molecular selective device (26) to the well (14);
removing the molecular selective device (26) from the substrate (12); and
performing a sensing technique on the components in the well (14).

12. A surface enhanced Raman spectroscopy (SERS) sensing system (100), comprising:
the molecular sensing device (10) as defined in claim 1;
a sample containing a component to be filtered through the molecular selective device (26) and introduced into the well (14) of the molecular sensing device (10); and
a Raman reader operatively positioned with respect to the well (14) of the molecular sensing device (10).

13. A method for making a molecular sensing device (10), the method comprising:
forming a well (14) i) in a material (16) that is positioned on a surface (S₁₂) of a substrate (12) or ii) in the surface (S₁₂) of the substrate (12);
forming a signal amplifying structure (18) in the well the signal amplifying structure (18) to amplify a signal for one of: surface enhanced Raman spectroscopy; enhanced fluorescence; and enhanced chemiluminescence; and
removably positioning a molecular selective device (26) in operative contact with the well (14) the molecular selective device (26) configured to allow molecules of interest to pass to the well and to filter out background molecules.

14. The method as defined in claim 13 wherein removably positioning the molecular selective device (26) includes removably bonding a membrane (28) having a molecular weight threshold to the substrate (12) so that the membrane (28) covers the well (14).

15. The method as defined in claim 13 wherein removably positioning the molecular selective device (26) includes placing a solid extraction column (30) on the substrate (12) so that a molecular extraction material (34) packed within a housing (32) of the solid extraction column (30) is spaced from and positioned over the well (14).

## Patentansprüche

1. Molekularmessvorrichtung (10), Folgendes umfassend:
ein Substrat (12);
eine Vertiefung (14), i) ausgebildet in einem Material (16), das auf einer Oberfläche (S₁₂) des Substrats (12) angeordnet ist, oder ii) ausgebildet in einer Oberfläche (S₁₂) des Substrats (12);
eine Signalverstärkungsanordnung (18), angeordnet in der Vertiefung, wobei die Signalverstärkungsanordnung (18) dazu dient, ein Signal für eines der Folgenden zu verstärken: oberflächenverstärkte Ramanspektroskopie; verstärkte Fluoreszenz; und verstärkte Chemilumineszenz; und
eine molekular selektive Vorrichtung (26), entfernbar mit der Vertiefung (14) wirkverbunden angeordnet, wobei die molekular selektive Vorrichtung (26) konfiguriert ist, Moleküle von Interesse zu der Vertiefung passieren zu lassen und Hintergrundmoleküle herauszufiltern.

2. Molekularmessvorrichtung (10) nach Anspruch 1, wobei die molekular selektive Vorrichtung (26) eine Membran (28) mit einem Molekülmassenschwellenwert ist.

3. Molekularmessvorrichtung (10) nach Anspruch 2, wobei die Membran (28) ausgewählt ist aus einer Cellulosemembran und einer porösen anorganischen Membran mit einer Dicke von höchstens 100 µm.

4. Molekularmessvorrichtung (10) nach Anspruch 2, wobei die Membran (28) entfernbar an Bereiche des Substrats (12) oder des Materials (16) neben der Vertiefung (14) gebunden ist.

5. Molekularmessvorrichtung (10) nach Anspruch 1, wobei die molekular selektive Vorrichtung (26) eine Feststoffextraktionssäule (30) ist, die Folgendes enthält:
ein Gehäuse (32); und
ein in das Gehäuse (32) gepacktes molekulares Extraktionsmaterial (34).

6. Molekularmessvorrichtung (10) nach Anspruch 5, ferner umfassend eine poröse Membran (36), mit dem Gehäuse (32) verbunden, um das molekulare Extraktionsmaterial (34) in dem Gehäuse (32) zu halten.

7. Molekularmessvorrichtung (10) nach Anspruch 5, wobei das molekulare Extraktionsmaterial (34) ausgewählt ist aus vernetzten Polymerkügelchen, Siliciumdioxidkügelchen, Glaskügelchen, Aluminiumoxidkügelchen, Polystyrolkügelchen, funktionalisierten Kügelchen oder Kombinationen daraus.

8. Molekularmessvorrichtung (10) nach Anspruch 1, ferner ein in die Vertiefung (14) eingebrachtes inertes Fluid (24) umfassend.

9. Molekularmessvorrichtung (10) nach Anspruch 1, wobei die Signalverstärkungsanordnung (18) eine Ramanspektroskopie-Verstärkungsanordnung ist.

10. Molekularmessvorrichtung (10) nach Anspruch 1, wobei die Vorrichtung Folgendes enthält:
eine Anordnung aus diskreten Vertiefungen (14), i) ausgebildet in dem Material (16), das auf der Oberfläche (S₁₂) des Substrats (12) angeordnet ist, oder ii) ausgebildet in der Oberfläche (S₁₂) des Substrats (12);
eine entsprechende Signalverstärkungsanordnung (18) in jeder der diskreten Vertiefungen (14); und
eine entsprechende molekular selektive Vorrichtung (26), entfernbar mit jeder der diskreten Vertiefungen (14) wirkverbunden angeordnet.

11. Verfahren zum Verwenden der Molekularmessvorrichtung (10) nach Anspruch 1, wobei das Verfahren Folgendes umfasst:
wenn die molekular selektive Vorrichtung (26) mit der Vertiefung (14) wirkverbunden angeordnet ist, Aussetzen der molekular selektiven Vorrichtung (26) gegenüber einer Probe, um durch die molekular selektive Vorrichtung (26) gefilterte Komponenten in die Vertiefung (14) einzubringen;
Entfernen der molekular selektiven Vorrichtung (26) von dem Substrat (12); und
Ausführen eines Messvorgangs an den Komponenten in der Vertiefung (14).

12. Messsystem (100) für oberflächenverstärkte Ramanspektroskopie (surface enhanced Raman spectroscopy - SERS), Folgendes umfassend:
die Molekularmessvorrichtung (10) nach Anspruch 1;
eine Probe, die eine durch die molekular selektive Vorrichtung (26) zu filternde und in die Vertiefung (14) der Molekularmessvorrichtung (10) einzubringende Komponente enthält;
und
ein Raman-Messgerät, funktionsfähig bezogen auf die Vertiefung (14) der Molekularmessvorrichtung (10) angeordnet.

13. Verfahren zum Herstellen einer Molekularmessvorrichtung (10), wobei das Verfahren Folgendes umfasst:
Ausbilden einer Vertiefung (14) i) in einem Material (16), das auf einer Oberfläche (S₁₂) eines Substrats (12) angeordnet ist, oder ii) in der Oberfläche (S₁₂) des Substrats (12);
Ausbilden einer Signalverstärkungsanordnung (18) in der Vertiefung, wobei die Signalverstärkungsanordnung (18) dazu dient, ein Signal für eines der Folgenden zu verstärken: oberflächenverstärkte Ramanspektroskopie; verstärkte Fluoreszenz; und verstärkte Chemilumineszenz; und
entfernbares Anordnen einer molekular selektiven Vorrichtung (26), mit der Vertiefung (14) wirkverbunden, wobei die molekular selektive Vorrichtung (26) konfiguriert ist, Moleküle von Interesse zu der Vertiefung passieren zu lassen und Hintergrundmoleküle herauszufiltern.

14. Verfahren nach Anspruch 13, wobei das entfernbare Anordnen der molekular selektiven Vorrichtung (26) ein entfernbares Anhaften einer Membran (28) mit einem Molekülmassenschwellenwert an das Substrat (12) derart umfasst, dass die Membran (28) die Vertiefung (14) abdeckt.

15. Verfahren nach Anspruch 13, wobei das entfernbare Anordnen der molekular selektiven Vorrichtung (26) ein Positionieren einer Feststoffextraktionssäule (30) auf dem Substrat (12) derart umfasst, dass ein in ein Gehäuse (32) der Feststoffextraktionssäule (30) gepacktes molekulares Extraktionsmaterial (34) von der Vertiefung (14) beabstandet und darüber angeordnet wird.

## Revendications

1. Un dispositif de détection moléculaire (10) comprenant :
un substrat (12) ;
un puits (14) i) formé dans un matériau (16) qui est positionné sur une surface (12) du substrat (12) ou ii) formé dans une surface (12) du substrat (12) ;
une structure d'amplification de signal (18) positionnée dans le puits, la structure d'amplification de signal (18) pour amplifier un signal destiné à l'un parmi : la spectroscopie Raman exaltée de surface ; la fluorescence améliorée ; et la chimiluminescence améliorée ; et
un dispositif moléculaire sélectif (26) positionné de manière amovible en contact fonctionnel avec le puits (14), le dispositif moléculaire sélectif (26) conçu pour permettre aux molécules d'intérêt de passer vers le puits et de filtrer les molécules de base.

2. Le dispositif de détection moléculaire (10), défini selon la revendication 1 dans lequel le dispositif moléculaire sélectif (26) est une membrane (28) présentant un seuil de poids moléculaire.

3. Le dispositif de détection moléculaire (10), défini selon la revendication 2, dans lequel la membrane (28) est choisie parmi une membrane de cellulose et une membrane inorganique poreuse présentant une épaisseur égale ou inférieure à 100 µm.

4. Le dispositif de détection moléculaire (10), défini selon la revendication 2, dans lequel la membrane (28) est liée de manière amovible à des zones du substrat (12) ou au matériau (16) adjacent au puits (14).

5. Le dispositif de détection moléculaire (10), défini selon la revendication 1, dans lequel le dispositif moléculaire sélectif (26) est une colonne d'extraction solide (30) comprenant :
un logement (32) ; et
un matériau d'extraction moléculaire (34) conditionné à l'intérieur du logement (32).

6. Le dispositif de détection moléculaire (10), défini selon la revendication 5, comprenant en outre une membrane poreuse (36) liée au logement (32) afin de maintenir le matériau d'extraction moléculaire (34) à l'intérieur du logement (32).

7. Le dispositif de détection moléculaire (10), défini selon la revendication 5, dans lequel le matériau d'extraction moléculaire (34) est choisi parmi des sphères de polymère réticulé, des sphères de silice, des sphères de verre, des sphères d'alumine, des sphères de polystyrène, des sphères fonctionnalisées ou des combinaisons de celles-ci.

8. Le dispositif de détection moléculaire (10), défini selon la revendication 1, comprenant en outre en un fluide inerte (24) incorporé dans le puits (14).

9. Le dispositif de détection moléculaire (10), défini selon la revendication 1, dans lequel la structure d'amplification de signal (18) est une structure de spectroscopie Raman exaltée.

10. Le dispositif de détection moléculaire (10), défini selon la revendication 1 dans lequel le dispositif comprend :
un réseau de puits distincts (14) i) formé dans le matériau (16) qui est positionné sur la surface (12) du substrat (12) ou ii) formé dans la surface (12) du substrat (12) ;
une structure d'amplification de signal respective (18) dans chacun des puits distincts (14) ; et un dispositif moléculaire sélectif respectif (26) positionné de manière amovible en contact fonctionnel avec chacun des puits distincts (14).

11. Un procédé destiné à l'utilisation du dispositif de détection moléculaire (10), défini selon la revendication 1, le procédé consistant à :
lorsque le dispositif moléculaire sélectif (26) est positionné en contact fonctionnel avec le puits (14), exposer le dispositif moléculaire sélectif (26) à un échantillon pour introduire les constituants filtrés à travers le dispositif moléculaire sélectif (26) vers le puits (14) ;
retirer le dispositif moléculaire sélectif (26) du substrat (12) ; et mettre en oeuvre une technique de détection sur les constituants dans le puits (14).

12. Un système de détection par spectroscopie Raman exaltée de surface (SERS) (100), comprenant :
le dispositif de détection moléculaire (10), défini selon la revendication 1 ;
un échantillon contenant un constituant devant être filtré à travers le dispositif moléculaire sélectif (26) et introduit dans le puits (14) du dispositif de détection moléculaire (10) ; et
un lecteur de Raman positionné de manière fonctionnelle par rapport au puits (14) du dispositif de détection moléculaire (10).

13. Un procédé destiné à fabriquer un dispositif de détection moléculaire (10), le procédé consistant à :
former un puits (14) i) dans un matériau (16) qui est positionné sur une surface (12) d'un substrat (12) ou ii) formé dans la surface (12) du substrat (12) ;
former une structure d'amplification de signal (18) dans le puits, la structure d'amplification de signal (18) pour amplifier un signal destiné à l'un parmi : la spectroscopie Raman exaltée de surface ; la fluorescence améliorée ; et la chimiluminescence améliorée ; et
positionner de manière amovible un dispositif moléculaire sélectif (26) en contact fonctionnel avec le puits (14), le dispositif moléculaire sélectif (26) conçu pour permettre aux molécules d'intérêt de passer vers le puits et de filtrer les molécules de base.

14. Le procédé défini selon la revendication 13, dans lequel le positionnement de manière amovible du dispositif moléculaire sélectif (26) comprend le collage de manière amovible d'une membrane (28) présentant un seuil de poids moléculaire par rapport au substrat (12) de manière à ce que la membrane (28) couvre le puits (14).

15. Le procédé défini selon la revendication 13, dans lequel le positionnement de manière amovible du dispositif moléculaire sélectif (26) comprend le placement d'une colonne d'extraction solide (30) sur le substrat (12) de manière à ce qu'un matériau d'extraction moléculaire (34) conditionné à l'intérieur du logement (32) de la colonne d'extraction solide (30) soit espacé et positionné au-dessus du puits (14).
